# EUROPEAN PATENT SPECIFICATION

(11) **EP 2 413 805 B1**
(45) Date of publication and mention of the grant of the patent: **12.02.2020**
(21) Application number: 10728462.2
(22) Date of filing: 24.03.2010
(51) Int. Cl.: A61B 8/12

(54) **INTRAVASCULAR IMAGING SYSTEMS WITH MULTIPLE PULLBACK RATES**
INTRAVASKULÄREN BILDGEBUNGSSYSTEME MIT MEHREREN PULLBACK-RATEN
SYSTÈMES D'IMAGERIE INTRAVASCULAIRE AVEC DE MULTIPLES VITESSES DE RETRAIT

(30) Priority: 31.03.2009 US 415807
(43) Date of publication of application: 08.02.2012
(73) Proprietor: Boston Scientific Scimed, Inc., Maple Grove, MN 55311 (US)
(72) Inventor: KNIGHT, Jon, M., Pleasanton, CA 94588 (US)
(74) Representative: Vossius & Partner Patentanwälte Rechtsanwälte mbB
(86) International application number: PCT/US2010/028442
(87) International publication number: WO 2010/117632

(56) References cited:
- WO-A2-2007/103726
- US-A1- 2006 052 700
- SPIE, PO BOX 10 BELLINGHAM WA 98227-0010 USA, 2 October 2007 (2007-10-02), XP040247973

## Description

### TECHNICAL FIELD

The present invention is directed to the area of intravascular imaging systems. The present invention is also directed to intravascular imaging systems configured and arranged to perform an intravascular imaging procedure using multiple linear rates pullback.

### BACKGROUND

Intravascular ultrasound ("IVUS") imaging systems have proven diagnostic capabilities for a variety of diseases and disorders. For example, IVUS imaging systems have been used as an imaging modality for diagnosing blocked blood vessels and providing information to aid medical practitioners in selecting and placing stents and other devices to restore or increase blood flow. IVUS imaging systems have been used to diagnose atheromatous plaque build-up at particular locations within blood vessels. IVUS imaging systems can be used to determine the existence of an intravascular obstruction or stenosis, as well as the nature and degree of the obstruction or stenosis. IVUS imaging systems can be used to visualize segments of a vascular system that may be difficult to visualize using other intravascular imaging techniques, such as angiography, due to, for example, movement (e.g., a beating heart) or obstruction by one or more structures (e.g., one or more blood vessels not desired to be imaged). IVUS imaging systems can be used to monitor or assess ongoing intravascular treatments, such as angiography and stent placement in real (or almost real) time. Moreover, IVUS imaging systems can be used to monitor one or more heart chambers.

IVUS imaging systems have been developed to provide a diagnostic tool for visualizing a variety of diseases or disorders. An IVUS imaging system can include a control module (with a pulse generator, an image processor, and a monitor), a catheter, and one or more transducers disposed in the catheter. The transducer-containing catheter can be positioned in a lumen or cavity within, or in proximity to, a region to be imaged, such as a blood vessel wall or patient tissue in proximity to a blood vessel wall. The pulse generator in the control module generates electrical pulses that are delivered to the one or more transducers and transformed to acoustic pulses that are transmitted through patient tissue. Reflected pulses of the transmitted acoustic pulses are absorbed by the one or more transducers and transformed to electric pulses. The transformed electric pulses are delivered to the image processor and converted to an image displayable on the monitor.

Optical Coherence Tomography ("OCT") is another imaging modality with proven capabilities for use in diagnosing intravasculature diseases and disorders. OCT uses optical signals to image patient tissue. Optical signals emitted from an OCT system are reflected from patient tissue and collected and processed by the OCT system to form an image.

US 2006/052700 A1 discusses a pressure measurement system, comprising a pressure detection device with a pressure sensor guidewire connected to an electrical carrier for transmitting pressure data from the sensor to a processing means. The guidewire is inserted into a vessel of a subject and an image data representative of the vessel is captured. The processing means includes computer executable instruction for manipulating image data and pressure data to generate an output in which the pressure data is mapped onto a corresponding position on an image where that pressure data was detected to provide an integrated graphical image output on a monitoring means.

Furthermore, a system is discussed in Gardner et al., "A catheter-based near-infrared scanning spectroscopy system for imaging lipid-rich plaques in human coronary arteries in vivo", Proceedings of SPIE, vol. 6765, 2007, which is a scanning, near-infrared, optical-fiber-based, spectroscopic cardiac catheter system capable of acquiring NIR reflectance spectra from coronary arteries through flowing blood under automated pullback and rotation in order to identify lipid-rich plaques.

In WO 2007/103726 A2 techniques for imaging a blood vessel are described. A transducer attached to a catheter is guided to a first site within a blood vessel. The catheter is moved to a second site proximal to the first site at a speed selected to enable the transducer to generate a signal indicative of a geometry of the wall, the lumen, and a portion of the side branch. A first image of the blood vessel obtained from the signal is oriented with a second image of the blood vessel such that a first portion of the first image aligns with a second portion of the second image.

### BRIEF SUMMARY

In one embodiment, a computer-readable medium includes processor-executable instructions for imaging tissue. The processor-executable instructions, when installed onto an intravascular imaging system, enable the system to perform actions. The actions include imaging a survey region of patient vasculature with an intravascular imager to obtain a set of first images while pulling back the imager from the first end of the survey region to a second end of the survey region opposite the first end at a first linear rate of pullback. The actions also include imaging a
region of interest identified within the survey region to obtain a set of second images while pulling back the imager from a first end of the region of interest to a second end of the region of interest opposite the first end. During imaging of the region of interest, the imager is pulled back at a second linear rate of pullback that is less than the first linear rate of pullback. The actions also include displaying at least a portion of the set of second images.

In another embodiment, an intravascular imaging system includes a catheter and a control module, wherein at least one imager is disposed in the catheter that is insertable into patient vasculature. The catheter is coupled to the control module. The intravascular imaging system also includes a pulse generator, a motor, and a processor, which are disposed in the control module. The processor is for executing processor-readable instructions that enable actions. The actions include imaging a survey region of patient vasculature to obtain a set of first images while pulling back the imager from the first end of the survey region to a second end of the survey region opposite the first end at a first linear rate of pullback. The actions also include imaging a region of interest identified within the survey region to obtain a set of second images while pulling back the imager from a first end of the region of interest to a second end of the region of interest opposite the first end. During imaging of the region of interest, the imager is pulled back at a second linear rate of pullback that is less than the first linear rate of pullback. The actions also include displaying at least a portion of the set of second images.

### BRIEF DESCRIPTION OF THE DRAWINGS

Non-limiting and non-exhaustive embodiments of the present invention are described with reference to the following drawings. In the drawings, like reference numerals refer to like parts throughout the various figures unless otherwise specified.

For a better understanding of the present invention, reference will be made to the following Detailed Description, which is to be read in association with the accompanying drawings, wherein:
FIG. 1 is a schematic view of one embodiment of an intravascular ultrasound imaging system, according to the invention;
FIG. 2 is a schematic side view of one embodiment of a catheter of an intravascular ultrasound imaging system, according to the invention;
FIG. 3 is a schematic perspective view of one embodiment of a distal end of the catheter shown in FIG. 2 with an imaging core disposed in a lumen defined in the catheter, according to the invention;
FIG. 4 is a schematic longitudinal cross-sectional view of one embodiment of a portion of a catheter extending along a portion of a patient blood vessel having a plaque in a wall of the blood vessel, according to the invention;
FIG. 5A is a schematic representation of one embodiment of a set of images formed from a survey pullback of an imaging procedure, a marker in proximity to some of the images marks a region of interest contained on those images, according to the invention;
FIG. 5B is a schematic representation of one embodiment of a set of images formed from a survey pullback of an imaging procedure, markers in proximity to some of the images mark the end points of a region of interest contained on those images, according to the invention;
FIG. 6 is a schematic representation of one embodiment of the set of images formed from the survey pullback of FIG. 5A, another set of images formed from an inspection pullback is formed along the region of interest of FIG. 5A, according to the invention; and
FIG. 7 is a flow diagram showing one exemplary embodiment of an imaging procedure using an intravascular imaging system with multiple pullback rates, according to the invention.

### DETAILED DESCRIPTION

The present invention is directed to the area of intravascular imaging systems. The present invention is also directed to intravascular imaging systems configured and arranged to perform an intravascular imaging procedure using multiple linear rates of pullback, as well as methods of making and using the intravascular ultrasound systems.

The methods, systems, and devices described herein may be embodied in many different forms and should not be construed as limited to the embodiments set forth herein. Accordingly, the methods, systems, and devices described herein may take the form of an entirely hardware embodiment, an entirely software embodiment or an embodiment combining software and hardware aspects. The methods described herein can be performed using any type of computing device, such as a computer, that includes a processor or any combination of computing devices where each device performs at least part of the process.

Suitable computing devices typically include mass memory and typically include communication between devices. The mass memory illustrates a type of computer-readable media, namely computer storage media. Computer storage media may include volatile, nonvolatile, removable, and non-removable media implemented in any method or technology for storage of information, such as computer readable instructions, data structures, program modules, or other data. Examples of computer storage media include RAM, ROM, EEPROM, flash memory, or other memory technology, CD-ROM, digital versatile disks (DVD) or other optical storage, magnetic cassettes, magnetic tape, magnetic disk storage or other magnetic storage devices, or any other medium which can be used to store the desired information and which can be accessed by a computing device.

Methods of communication between devices or components of a system can include both wired and wireless (e.g., RF, optical, or infrared) communications methods and such methods provide another type of computer readable media; namely communication media. Communication media typically embodies computer-readable instructions, data structures, program modules, or other data in a modulated data signal such as a carrier wave, data signal, or other transport mechanism and include any information delivery media. The terms "modulated data signal," and "carrier-wave signal" includes a signal that has one or more of its characteristics set or changed in such a manner as to encode information, instructions, data, and the like, in the signal. By way of example, communication media includes wired media such as twisted pair, coaxial cable, fiber optics, wave guides, and other wired media and wireless media such as acoustic, RF, infrared, and other wireless media.

Suitable intravascular ultrasound ("IVUS") imaging systems include, but are not limited to, one or more transducers disposed on a distal end of a catheter configured and arranged for percutaneous insertion into a patient. Examples of IVUS imaging systems with catheters are found in, for example, U.S. Patents Nos. 7,306,561; and 6,945,938; as well as U.S. Patent Application Publication Nos. 20060253028; 20070016054; 20070038111; 20060173350; and 200601005 22.

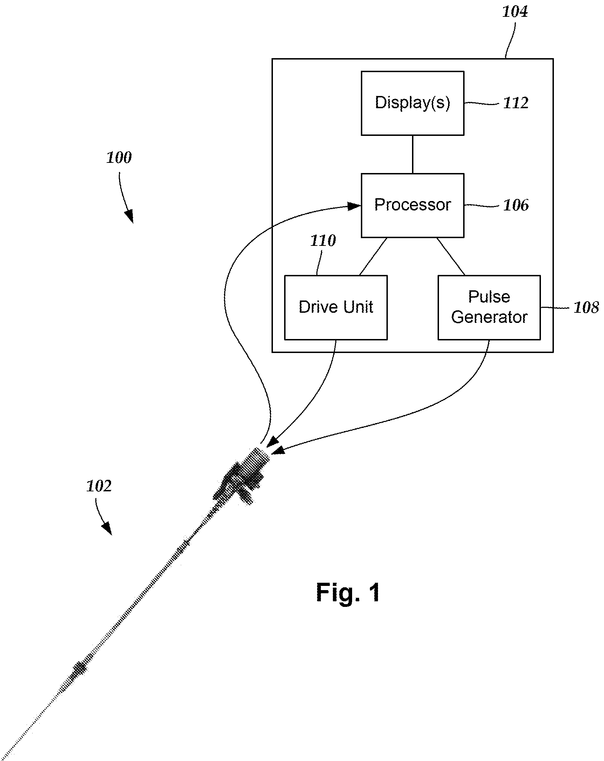

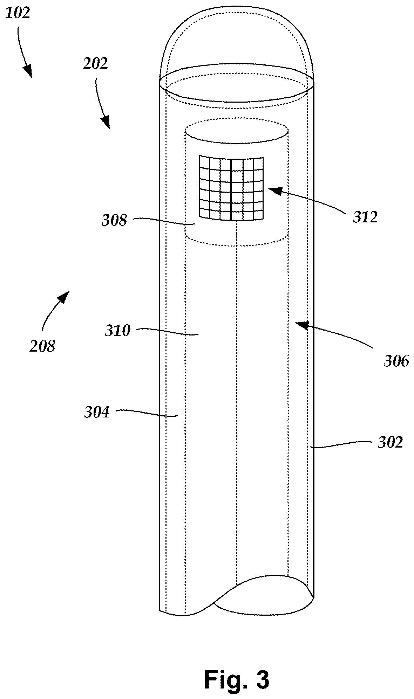

Figure 1 illustrates schematically one embodiment of an IVUS imaging system 100. The IVUS imaging system 100 includes a catheter 102 that is coupleable to a control module 104. The control module 104 may include, for example, a processor 106, a pulse generator 108, a motor 110, and one or more displays 112. In at least some embodiments, the pulse generator 108 forms electric pulses that may be input to one or more transducers (312 in Figure 3) disposed in the catheter 102. In at least some embodiments, mechanical energy from the motor 110 may be used to drive an imaging core (306 in Figure 3) disposed in the catheter 102. In at least some embodiments, electric pulses transmitted from the one or more transducers (312 in Figure 3) may be input to the processor 106 for processing. In at least some embodiments, the processed electric pulses from the one or more transducers (312 in Figure 3) may be displayed as one or more images on the one or more displays 112. In at least some embodiments, the processor 106 may also be used to control the functioning of one or more of the other components of the control module 104. For example, the processor 106 may be used to control at least one of the frequency or duration of the electrical pulses transmitted from the pulse generator 108, the rotation rate of the imaging core (306 in Figure 3) by the motor 110, the velocity or length of the pullback of the imaging core (306 in Figure 3) by the motor 110, or one or more properties of one or more images formed on the one or more displays 112.

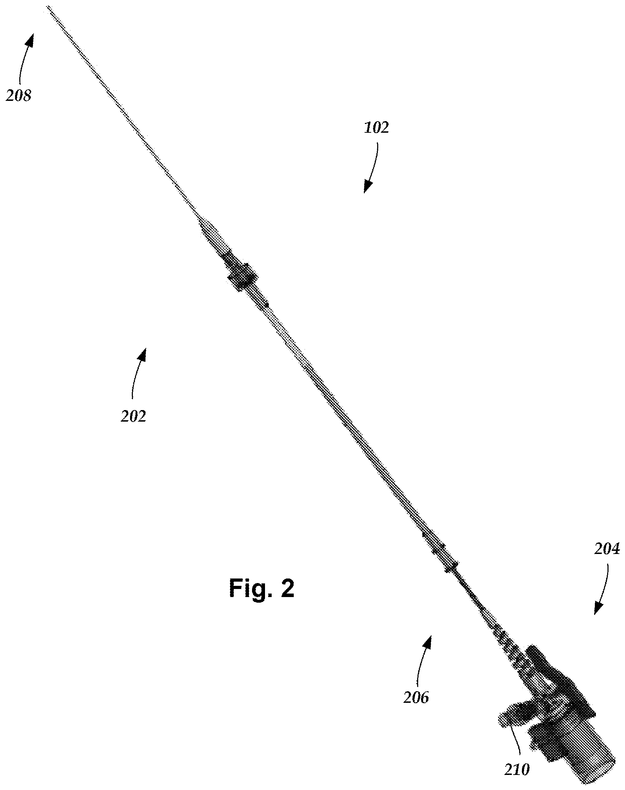

Figure 2 is a schematic side view of one embodiment of the catheter 102 of the IVUS imaging system (100 in Figure 1). The catheter 102 includes an elongated member 202 and a hub 204. The elongated member 202 includes a proximal end 206 and a distal end 208. In Figure 2, the proximal end 206 of the elongated member 202 is coupled to the catheter hub 204 and the distal end 208 of the elongated member is configured and arranged for percutaneous insertion into a patient. In at least some embodiments, the catheter 102 defines at least one flush port, such as flush port 210. In at least some embodiments, the flush port 210 is defined in the hub 204. In at least some embodiments, the catheter 102 does not use a flush port 204. In at least some embodiments, the hub 204 is configured and arranged to couple to the control module (104 in Figure 1). In some embodiments, the elongated member 202 and the hub 204 are formed as a unitary body. In other embodiments, the elongated member 202 and the catheter hub 204 are formed separately and subsequently assembled together.

Figure 3 is a schematic perspective view of one embodiment of the distal end 208 of the elongated member 202 of the catheter 102. The elongated member 202 includes a sheath 302 and a lumen 304. An imaging core 306 is disposed in the lumen 304. The imaging core 306 includes an imaging device 308 coupled to a distal end of a rotatable driveshaft 310.

The sheath 302 may be formed from any flexible, biocompatible material suitable for insertion into a patient. Examples of suitable materials include, for example, polyethylene, polyurethane, plastic, spiral-cut stainless steel, nitinol hypotube, and the like or combinations thereof.

One or more transducers 312 may be mounted to the imaging device 308 and employed to transmit and receive acoustic pulses. In a preferred embodiment (as shown in Figure 3), an array of transducers 312 are mounted to the imaging device 308. In other embodiments, a single transducer may be employed. In yet other embodiments, multiple transducers in an irregular-array may be employed. Any number of transducers 312 can be used. For example, there can be two, three, four, five, six, seven, eight, nine, ten, twelve, fifteen, sixteen, twenty, twenty-five, fifty, one hundred, five hundred, one thousand, or more transducers. As will be recognized, other numbers of transducers may also be used. In at least some embodiments, the one or more transducers 312 are configured into an annular arrangement. In at least some embodiments, the one or more transducers 312 are fixed in place and do not rotate.

The one or more transducers 312 may be formed from one or more known materials capable of transforming applied electrical pulses to pressure distortions on the surface of the one or more transducers 312, and vice versa. Examples of suitable materials include piezoelectric ceramic materials, piezocomposite materials, piezoelectric plastics, barium titanates, lead zirconate titanates, lead metaniobates, polyvinylidenefluorides, and the like. Other transducer technologies include composite materials, single-crystal composites, and semiconductor devices (*e.g.,* capacitive micromachined ultrasound transducers ("cMUT"), piezoelectric micromachined ultrasound transducers ("pMUT"), or the like)
The pressure distortions on the surface of the one or more transducers 312 form acoustic pulses of a frequency based on the resonant frequencies of the one or more transducers 312. The resonant frequencies of the one or more transducers 312 may be affected by the size, shape, and material used to form the one or more transducers 312. The one or more transducers 312 may be formed in any shape suitable for positioning within the catheter 102 and for propagating acoustic pulses of a desired frequency in one or more selected directions. For example, transducers may be disc-shaped, block-shaped, rectangular-shaped, oval-shaped, and the like. The one or more transducers may be formed in the desired shape by any process including, for example, dicing, dice and fill, machining, microfabrication, and the like.

As an example, each of the one or more transducers 312 may include a layer of piezoelectric material sandwiched between a conductive acoustic lens and a conductive backing material formed from an acoustically absorbent material (*e.g.,* an epoxy substrate with tungsten particles). During operation, the piezoelectric layer may be electrically excited by both the backing material and the acoustic lens to cause the emission of acoustic pulses.

In at least some embodiments, the one or more transducers 312 can be used to form a radial cross-sectional image of a surrounding space. Thus, for example, when the one or more transducers 312 are disposed in the catheter 102 and inserted into a blood vessel of a patient, the one more transducers 312 may be used to form an image of the walls of the blood vessel and tissue surrounding the blood vessel.

In at least some embodiments, the imaging core 306 may be rotated about a longitudinal axis of the catheter 102. As the imaging core 306 rotates, the one or more transducers 312 emit acoustic pulses in different radial directions. When an emitted acoustic pulse with sufficient energy encounters one or more medium boundaries, such as one or more tissue boundaries, a portion of the emitted acoustic pulse is reflected back to the emitting transducer as an echo pulse. Each echo pulse that reaches a transducer with sufficient energy to be detected is transformed to an electrical signal in the receiving transducer. The one or more transformed electrical signals are transmitted to the control module (104 in Figure 1) where the processor 106 processes the electrical-signal characteristics to form a displayable image of the imaged region based, at least in part, on a collection of information from each of the acoustic pulses transmitted and the echo pulses received. In at least some embodiments, the rotation of the imaging core 306 is driven by the motor 110 disposed in the control module (104 in Figure 1).

As the one or more transducers 312 rotate about the longitudinal axis of the catheter 102 emitting acoustic pulses, a plurality of images are formed that collectively form a radial cross-sectional image of a portion of the region surrounding the one or more transducers 312, such as the walls of a blood vessel of interest and the tissue surrounding the blood vessel. In at least some embodiments, the radial cross-sectional image can be displayed on one or more displays 112. In at least some embodiments, the one or more transducers 312 are fixed in place and do not rotate during an imaging procedure. In at least some embodiments, at least one of the imaging core 306 or the one or more transducers 312 are manually rotated.

In at least some embodiments, the imaging core 306 may also move longitudinally along the blood vessel within which the catheter 102 is inserted so that a plurality of cross-sectional images may be formed along a longitudinal length of the blood vessel. In at least some embodiments, during an imaging procedure the one or more transducers 312 may be retracted (*i.e.,* pulled back) along the longitudinal length of the catheter 102. In at least some embodiments, the catheter 102 includes at least one telescoping section that can be retracted during pullback of the one or more transducers 312. In at least some embodiments, the motor 110 drives the pullback of the imaging core 306 within the catheter 102. In at least some embodiments, the motor 110 pullback distance of the imaging core is at least 5 cm. In at least some embodiments, the motor 110 pullback distance of the imaging core is at least 10 cm. In at least some embodiments, the motor 110 pullback distance of the imaging core is at least 15 cm. In at least some embodiments, the motor 110 pullback distance of the imaging core is at least 20 cm. In at least some embodiments, the motor 110 pullback distance of the imaging core is at least 25 cm. In at least some embodiments, the entire catheter 102 can be retracted during an imaging procedure either with or without the imaging core 306 moving longitudinally independently of the catheter 102.

In at least some embodiments, when the imaging core 306 is retracted while rotating, the images collectively form a continuous spiral shape along a blood vessel. In at least some embodiments, when the imagine core 306 is retracted while rotating, a stepper motor may be used to pull back the imaging core 306. The stepper motor can pull back the imaging core 306 a short distance and stop long enough for the one or more transducers 306 to capture an image before pulling back the imaging core 306 another short distance and again capturing another image, and so on, either with or without being rotated.

The quality of an image produced at different depths from the one or more transducers 312 may be affected by one or more factors including, for example, bandwidth, transducer focus, beam pattern, as well as the frequency of the acoustic pulse. The frequency of the acoustic pulse output from the one or more transducers 312 may also affect the penetration depth of the acoustic pulse output from the one or more transducers 312. In general, as the frequency of an acoustic pulse is lowered, the depth of the penetration of the acoustic pulse within patient tissue increases. In at least some embodiments, the IVUS imaging system 100 operates within a frequency range of 5 MHz to 100 MHz.

In at least some embodiments, one or more conductors 314 electrically couple the transducers 312 to the control module 104 (see *e.g.,* Figure 1). In at least some embodiments, the one or more conductors 314 extend along a longitudinal length of the rotatable driveshaft 310.

In at least some embodiments, the catheter 102 with one or more transducers 312 mounted to the distal end 208 of the imaging core 308 may be inserted percutaneously into a patient via an accessible blood vessel, such as the femoral artery, at a site remote from the selected portion of the selected region, such as a blood vessel, to be imaged. The catheter 102 may then be advanced through the blood vessels of the patient to the selected imaging site, such as a portion of a selected blood vessel.

Intravascular imaging techniques (*e.g.,* IVUS, OCT, or the like) are commonly used to diagnose patient diseases and disorders. Intravascular diseases and disorders may occur either at discrete locations within patient vasculature or be distributed over a larger intravascular region. In at least some embodiments, an imaging procedure includes a pullback of an imager within a catheter along a longitudinal portion of patient vasculature. A set of adjacent images are captured at a particular linear pullback rate along the portion of the vasculature. The images are processed and displayed to a user. As an example, one particular IVUS system captures 30 images per second and has a linear pullback rate in the range of 0.5 mm/sec to 1.0 mm/sec. Thus, a pullback along 10 cm of vasculature takes 100 to 200 seconds and captures 3,000 to 6,000 images. Typically, the majority of the images and the time it takes to capture the images are associated with healthy portions of patient vasculature not significant to the given diagnosis.

Systems and methods of using intravascular imaging systems to assess patient vasculature are described. In at least some embodiments, an imaging procedure includes a survey pullback and an inspection pullback. During the survey pullback, images are captured over a portion of patient vasculature. The locations of one or more regions of interest ("ROI"), such as focal areas, identified during the survey pullback may be subsequently imaged during the inspection pullback. In at least some embodiments, the identified ROIs are marked prior to the inspection pullback.

During the inspection pullback, marked ROIs are re-located and re-imaged. In at least some embodiments, the survey pullback and the inspection pullback are performed at different linear pullback rates. In at least some embodiments, the linear pullback rate of the survey pullback is greater than the linear pullback rate of the inspection pullback. In at least some embodiments, the amount of time it takes to perform an imaging procedure using a survey pullback and an inspection pullback of a portion of the survey pullback is less than the amount of time it takes to perform an imaging procedure using a single pullback with a conventional intravascular imaging system.

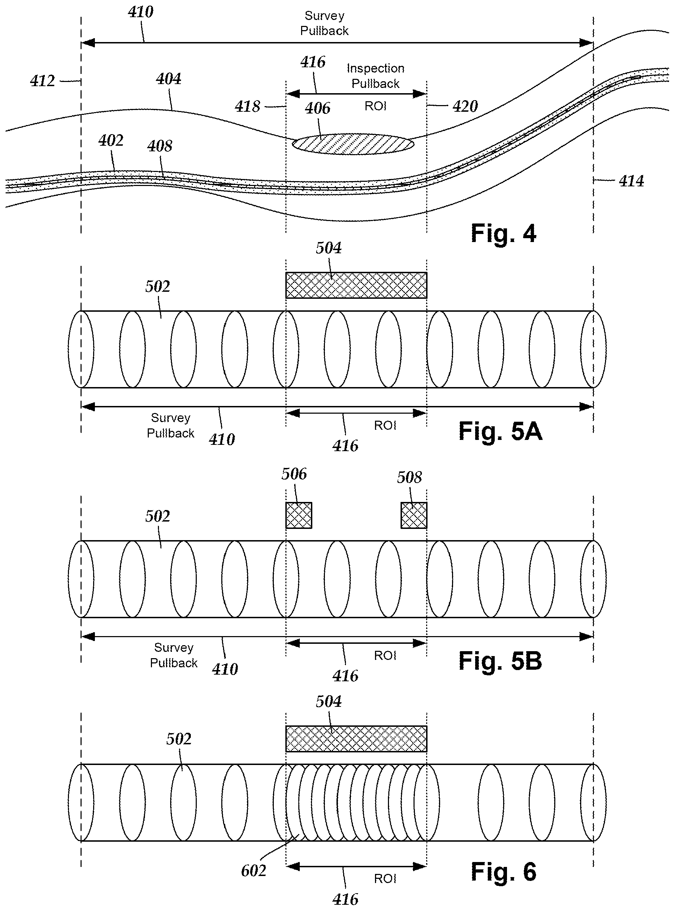

Figure 4 is a schematic longitudinal cross-sectional view of one embodiment of a portion of a catheter 402 extending along a portion of a patient blood vessel 404 having a plaque 406 in a wall of the blood vessel 404. The catheter 402 includes an imager 408 (*e.g.,* imaging core 306 of Figure 3) configured and arranged for imaging a survey region 410 of the blood vessel 404 bounded on a distal end by dashed line 412 and on a proximal end by dashed line 414. In at least some embodiments, the catheter 402 is held in a constant position while the imager 408 images the survey region 410 by pullback of the imager 408 within the catheter 402. In at least some embodiments, the survey region 410 is imaged at a first linear pullback rate.

When, during the pullback of the survey region 410, a ROI 416 (*e.g.,* the plaque 406, or the like) is identified, the imager 408 subsequently images just the ROI 416. In Figure 4, the ROI 416 is shown as the plaque 406 and the region of the blood vessel 404 flanking the plaque 406. The ROI 416 is bounded on a distal end by dotted line 418 and on a proximal end by dotted line 420. In at least some embodiments, the ROI 416 is re-imaged (*i.e.,* inspected) at a second linear rate of pullback that is different from the linear rate of the survey pullback.

The survey pullback captures a set of images of the blood vessel 404. The set of images can be used to locate one or more ROIs for re-imaging at a different linear rate of pullback. The set of images captured during the survey pullback can include any number of images. The widths of the images is determined by the width of the imaging beam of the imager. The adjacent images can be separated from one another by any center-to-center distance. In at least some embodiments, the center-to-centerl distances are set such that adjacent images are overlapping. In at least some embodiments, the center-to-center distances are set such that adjacent images are non-overlapping. In some embodiments, the center-to-center distances are set such that all of the imaged length of the blood vessel 404 is imaged. In other embodiments, the center-to-center distances are set such that portions of the blood vessel 404 between adjacent images are not imaged.

Figures 5A-5B are schematic representations of one embodiment of a set of images, such as image 502, formed during a pullback of the survey region 410. In Figures 5A-5B, the images are shown as adjacent cylinders abutting one another. In at least some embodiments, the set of images are processed by the control module (104 in Figure 1). In at least some embodiments, the set of images is displayed on the one or more displays (112 of Figure 1). In other embodiments, the set of images is displayed on another device coupled to the imager (408 in Figure 4). In at least some embodiments, the survey pullback is automatically performed under the control of the control module (104 in Figure 1). In at least some embodiments, the survey pullback is performed by another device coupled to the intravascular imaging system (*e.g.,* the IVUS system 100 in Figure 1).

One or more ROIs may be identified in a variety of different ways. For example, in at least some embodiments, the ROIs are identified using software, such as tissue characterization software. Such software can identify regions containing, for example, normal tissue, necrotic tissue, calcified tissue, lipidic tissue, and fibrotic tissue. Additionally, software can be used to identify heterogeneous tissues (*e.g.,* fibrolipidic tissue, or the like), as well as blood and various forms of thrombus. It will be understood that the above-listed tissues (as well as blood) are merely exemplary. There are many different other possible tissue permutations that can be identified using software.

An ROI may be selected to be a significant amount of non-normal tissue (*e.g.,* lipidic tissue). In at least some embodiments, ROIs are identified manually by a health care provider. For example, a health care provider may look at one or more displayed images captured during the survey pullback of the survey region 410. In some embodiments, software identification and manual identification may both be used.

In some embodiments, when an ROI, such as ROI 416, is identified, the ROI 416 may be marked on a display. In other embodiments, the identified ROI 416 may be marked internally by software. When the ROI 416 is marked on a display, the marker 504 is positioned on the display of the set of images in proximity to the location of the ROI 416 (*e.g.,* above the ROI, below the ROI, to the side of the ROI, over top of the ROI). In at least some embodiments, the marker 504 is automatically shown on a display by the control module (104 in Figure 1). In at least some embodiments, the marker 504 is applied to the display by a user of an intravascular imaging system. In at least some embodiments, at least one of the size or the location of the marker 504 can be adjusted by the user via the control module (104 in Figure 1). In at least some embodiments, as shown in Figure 5B, a plurality of markers 506 and 508 can be used to mark the ROI 416 in lieu of a single marker (504 in Figure 5A). In at least some embodiments, the markers 506 and 508 are positioned at the distal and proximal ends, respectfully, of the ROI 416.

In at least some embodiments, the survey region 410 is imaged using a linear pullback rate that is greater than the linear pullback rate used while imaging the ROI 416. In at least some embodiments, the survey region 410 is imaged using a linear pullback rate of no less than 2 mm/scc. In at least some embodiments, the survey region 410 is imaged using a linear pullback rate of no less than 5 mm/sec. In at least some embodiments, the survey region 410 is imaged using a linear pullback rate of no less than 10 mm/sec. In at least some embodiments, the survey region 410 is imaged using a linear pullback rate of no less than 15 mm/sec. In at least some embodiments, the survey region 410 is imaged using a linear pullback rate of no less than 20 mm/sec. In at least some embodiments, the survey region 410 is imaged using a linear pullback rate of no less than 30 mm/sec. In at least some embodiments, the survey region 410 is imaged using a linear pullback rate of no less than 40 mm/sec.

Once the ROI 416 is marked, the imager (408 in Figure 4) can be positioned at the distal end of the ROI 416. In some embodiments, the imager is automatically re-positioned at the distal end of the ROI 146, as marked on a display of the set of images of the survey region (410 in Figure 4) by one or more markers. In other embodiments, the imager is manually re-positioned at the distal end of the ROI 416 by a user of an intravascular imaging system. Once the imager is positioned at the distal end of the ROI 416, the imager can perform an inspection pullback from the distal end to the proximal end of the ROI 416. In at least some embodiments, the set of images captured by the inspection pullback include non-overlapping adjacent images. In at least some embodiments, the non-overlapping adjacent images abut one another such that there are no gaps between adjacent images. In preferred embodiments, the adjacent images overlap one another. It may be an advantage to have adjacent images overlay to ensure that there are no gaps between adjacent images and also to allow the control module (104 in Figure 1) to perform one or more image processing algorithms (*e.g.,* correlation, or the like) on the data from the images.

Figure 6 is a schematic representation of one embodiment of a set of images, such as image 602, formed from an inspection pullback along the ROI 416. In Figure 6, the set of overlapping images from the inspection pullback is overlaid onto the set of images, such as image 502, formed during the survey pullback of Figure 5A. In Figure 6, the center-to-center distance between adjacent images captured during the inspection pullback is less than the center-to-center distance between adjacent images captured during the survey pullback.

In at least some embodiments, the survey pullback is automatically performed under the control of the control module (104 in Figure 1). In at least some embodiments, the inspection pullback is performed by another device coupled to the intravascular imaging system (*e.g.,* the IVUS system 100 in Figure 1). In at least some embodiments, the imaging of the ROI 416 is performed at a linear pullback rate of no greater than 2 mm/sec.

In preferred embodiments, the images obtained during the inspection pullback are displayed. In at least some embodiments, only the images obtained during the inspection pullback are displayed. In at least some embodiments, the images obtained during the survey pullback and the images obtained during the inspection pullback are both displayed. In at least some embodiments, the display of the set of images from the inspection pullback are combined with the set of images from the survey pullback to form a composite image. In at least some embodiments, displayed images can be edited (*e.g.,* cropped, filtered, or the like).

As discussed above, with conventional intravascular imaging techniques, a 10 cm pullback may take 100 to 200 seconds. In at least some embodiments, when the survey region has a longitudinal length of 10 cm and is imaged at a linear pullback rate of 40 mm/sec, the survey pullback is performed in no more than 2.5 seconds. In at least some embodiments, when the ROI has a longitudinal length of 1 cm and is imaged at a linear pullback rate of 1 mm/sec, the inspection pullback is performed in no more than 10 seconds. Thus, even allowing for 30 seconds for placing the one or more markers in proximity to the ROI and re-positioning the imager to the distal end of the ROI, the imaging procedure takes no more than 42.5 seconds, as compared to 100 to 200 seconds for conventional methods.

Additionally, as also described above, with conventional intravascular imaging techniques, a 10 cm pullback may capture 3,000 to 6,000 images. Moreover, the majority of the images and the time it takes to capture the images are associated with healthy portions of patient vasculature that are not significant to the given diagnosis. In at least some embodiments, the size of the data stored on the control module (104 in Figure 1) can be reduced by performing a survey pullback and an inspection pullback. For example, in at least some embodiments, at an imaging rate of 30 images per second, the number of frames stored during the survey pullback and the inspection pullback, respectively, is (2.5 seconds x 30 images per second) + (10 seconds x 30 images per second) = 375 images, as compared to 3,000 to 6,000 images for conventional methods.

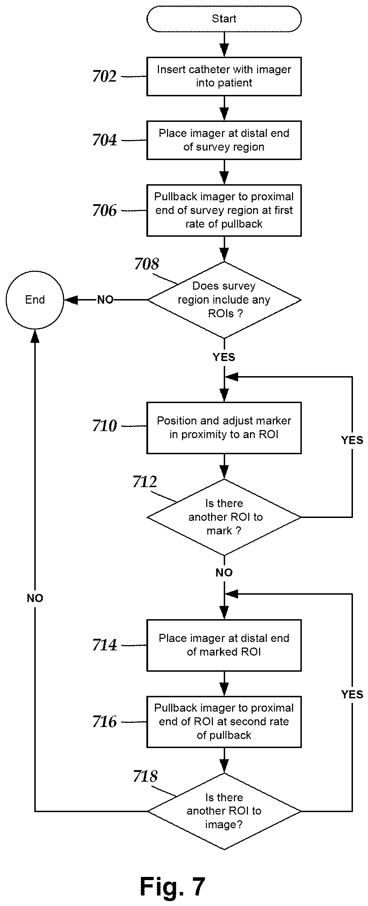

Figure 7 is a flow diagram showing one exemplary embodiment of an imaging procedure using an intravascular imaging system with multiple pullback rates. In step 702, a catheter with an imager is inserted into patient vasculature. In step 704, the imager is positioned at a distal end of a survey region. In step 706, the imager is pulled back along the survey region to a proximal end of the survey region at a first linear rate of pullback. When, in step 708, the survey region does not include any ROIs, the imaging procedure ends. Otherwise, in step 710 one or more markers are positioned (and adjusted, if applicable) in proximity to a ROI identified during the survey pullback. When, in step 712, the survey region includes one or more additional ROIs, control is passed back to step 710. Otherwise, in step 714 the imager is positioned at the distal end of a marked ROI. In step 716, the imager is pulled back along the ROI to a proximal end of the ROI at a second linear rate of pullback that is different than the first rate of linear pullback. When, in step 718, the survey region includes one or more additional marked ROIs, control is passed back to step 714. Otherwise, the imaging procedure ends.

It will be understood that each block of the flowchart illustrations, and combinations of blocks in the flowchart illustrations, as well any portion of the tissue classifier, imager, control module, systems and methods disclosed herein, can be implemented by computer program instructions. These program instructions may be provided to a processor to produce a machine, such that the instructions, which execute on the processor, create means for implementing the actions specified in the flowchart block or blocks or described for the tissue classifier, imager, control module, systems and methods disclosed herein. The computer program instructions may be executed by a processor to cause a series of operational steps to be performed by the processor to produce a computer implemented process. The computer program instructions may also cause at least some of the operational steps to be performed in parallel. Moreover, some of the steps may also be performed across more than one processor, such as might arise in a multi-processor computer system. In addition, one or more processes may also be performed concurrently with other processes, or even in a different sequence than illustrated without departing from the scope or spirit of the invention.

The computer program instructions can be stored on any suitable computer-readable medium including, but not limited to, RAM, ROM, EEPROM, flash memory or other memory technology, CD-ROM, digital versatile disks (DVD) or other optical storage, magnetic cassettes, magnetic tape, magnetic disk storage or other magnetic storage devices, or any other medium which can be used to store the desired information and which can be accessed by a computing device.

It will be understood that pullback along one or more of the survey region or the ROI may be performed by pulling the imager from a proximal end to a distal end of the region being imaged. It will also be understood that the intravascular imaging techniques described above can also be used with other types of imaging techniques that use a catheter insertable into patient vasculature. For example, the intravascular imaging techniques can be used with any imaging techniques configured and arranged to asses one or more measurable characteristics of patient tissue (*e.g.,* intravascular magnetic resonance imaging, spectroscopy, temperature mapping, or the like).

The above specification, examples and data provide a description of the manufacture and use of the composition of the invention.

## Claims

1. An intravascular imaging system (100) comprising:
a control module (104) including a pulse generator (108), a motor (110), and a processor (106);
a catheter (102, 402) coupleable to the control module (104); and
at least one imager (306, 408) disposed in the catheter (102, 402);
wherein the processor (106) is adapted for executing processor-readable instructions that enable actions, including:
pulling back the imager (306, 408) from a first end of a survey region (412) to a second end of the survey region (414) opposite the first end, wherein the imager (306, 408) is pulled back at a first linear rate of pullback;
imaging the survey region (410) to obtain a set of first images while pulling back the imager (306, 408) from the first end of the survey region (412) to the second end of the survey region (414);
identifying a region of interest (416) within the survey region (410) using at least a portion of the set of first images of the survey region; pulling back the imager (306, 408) from a first end of the region of interest (418) to a second end of the region of interest (420) opposite the first end, wherein the imager (306, 408) is pulled back at a second linear rate of pullback that is less than the first linear rate of pullback;
imaging the region of interest (416) identified within the survey region (410) to obtain a set of second images while pulling back the imager (306, 408) from a first end of the region of interest (418) to a second end of the region of interest (420) opposite the first end;
and
displaying at least a portion of the set of second images.

2. The intravascular imaging system (100) of claim 1, wherein the actions further comprise positioning the imager (306, 408) at the first end of the identified region of interest (418).

3. The intravascular imaging system (100) of any one of claims 1-2,
wherein the
actions further comprise displaying at least a portion of the set of first images.

4. A computer-readable medium having processor-executable instructions for imaging tissue and controlling at least one of rotation rate, velocity, or length of pullback of an imager (306,408), the processor-executable instructions when installed onto an intravascular imaging system (100) according to claim 1 enable the system to perform actions, comprising:
pulling back an imager (306, 408) from a first end of a survey region (412) to a second end of the survey region (414) opposite the first end, wherein the imager (306, 408) is pulled back at a first linear rate of pullback;
imaging a survey region (410) with the imager (306, 408) to obtain a set of first images while pulling back the imager (306, 408) from the first end of the survey region (412) to the second end of the survey region (414);
identifying a region of interest (416) within the survey region (410) using at least a portion of the set of first images of the survey region;
pulling back the imager (306, 408) from a first end of the region of interest (418) to a second end of the region of interest (420) opposite the first end, wherein the imager (306, 408) is pulled back at a second linear rate of pullback that is less than the first linear rate of pullback;
imaging the region of interest (416) identified within the survey region (410) to obtain a set of second images while pulling back the imager (306, 408) from a first end of the region of interest (418) to a second end of the region of interest (420) opposite the first end;
and
displaying at least a portion of the set of second images.

5. The computer-readable medium of claim 4,
wherein-the actions
further comprise positioning the imager (306, 408) at the first end of the identified region of interest (418).

6. The computer-readable medium of any one of claims 4-5,
wherein the actions
further comprise displaying at least a portion of the set of first images.

7. The computer-readable medium of claim 6,
wherein the actions further comprise
marking the identified region of interest (416) on the displayed set of first images.

## Patentansprüche

1. Intravaskuläres Abbildungssystem (100), das aufweist:
ein Steuermodul (104) mit einem Impulsgenerator (108), einem Motor (110) und einem Prozessor (106);
einen Katheter (102, 402), der mit dem Steuermodul (104) koppelbar ist; und
mindestens einen Bildwandler (306, 408), der im Katheter (102, 402) angeordnet ist;
wobei der Prozessor (106) zum Ausführen prozessorlesbarer Befehle geeignet ist, die Aktionen ermöglichen, darunter:
Zurückziehen des Bildwandlers (306, 408) von einem ersten Ende eines Betrachtungsbereichs (412) zu einem zweiten Ende des Betrachtungsbereichs (414) entgegengesetzt zum ersten Ende, wobei der Bildwandler (306, 408) mit einer ersten linearen Rückzugsgeschwindigkeit zurückgezogen wird;
Abbilden des Betrachtungsbereichs (410), um einen Satz erster Bilder zu erhalten, während der Bildwandler (306, 408) vom ersten Ende des Betrachtungsbereichs (412) zum zweiten Ende des Betrachtungsbereichs (414) zurückgezogen wird;
Identifizieren eines interessierenden Bereichs (416) im Betrachtungsbereich (410) mit Hilfe mindestens eines Anteils des Satzes erster Bilder des Betrachtungsbereichs;
Zurückziehen des Bildwandlers (306, 408) von einem ersten Ende des interessierenden Bereichs (418) zu einem zweiten Ende des interessierenden Bereichs (420) entgegengesetzt zum ersten Ende, wobei der Bildwandler (306, 408) mit einer zweiten linearen Rückzugsgeschwindigkeit zurückgezogen wird, die kleiner als die erste lineare Rückzugsgeschwindigkeit ist;
Abbilden des im Betrachtungsbereich (410) identifizierten interessierenden Bereichs (416), um einen Satz zweiter Bilder zu erhalten, während der Bildwandler (306, 408) von einem ersten Ende des interessierenden Bereichs (418) zu einem zweiten Ende des interessierenden Bereichs (420) entgegengesetzt zum ersten Ende zurückgezogen wird; und
Anzeigen mindestens eines Anteils des Satzes zweiter Bilder.

2. Intravaskuläres Abbildungssystem (100) nach Anspruch 1, wobei die Aktionen ferner aufweisen: Positionieren des Bildwandlers (306, 408) am ersten Ende des identifizierten interessierenden Bereichs (418).

3. Intravaskuläres Abbildungssystem (100) nach einem der Ansprüche 1 bis 2, wobei die Aktionen ferner aufweisen: Anzeigen mindestens eines Anteils des Satzes erster Bilder.

4. Computerlesbares Medium mit prozessorausführbaren Befehlen zum Abbilden von Gewebe und Steuern von Drehgeschwindigkeit, Geschwindigkeit und/oder Länge des Rückzugs eines Bildwandlers (306, 408), wobei die prozessorausführbaren Befehle bei Installation in einem intravaskulären Abbildungssystem (100) nach Anspruch 1 dem System ermöglichen, Aktionen durchzuführen, darunter:
Zurückziehen eines Bildwandlers (306, 408) von einem ersten Ende eines Betrachtungsbereichs (412) zu einem zweiten Ende des Betrachtungsbereichs (414) entgegengesetzt zum ersten Ende, wobei der Bildwandler (306, 408) mit einer ersten linearen Rückzugsgeschwindigkeit zurückgezogen wird;
Abbilden eines Betrachtungsbereichs (410) mit dem Bildwandler (306, 408), um einen Satz erster Bilder zu erhalten, während der Bildwandler (306, 408) vom ersten Ende des Betrachtungsbereichs (412) zum zweiten Ende des Betrachtungsbereichs (414) zurückgezogen wird;
Identifizieren eines interessierenden Bereichs (416) im Betrachtungsbereich (410) mit Hilfe mindestens eines Anteils des Satzes erster Bilder des Betrachtungsbereichs;
Zurückziehen des Bildwandlers (306, 408) von einem ersten Ende des interessierenden Bereichs (418) zu einem zweiten Ende des interessierenden Bereichs (420) entgegengesetzt zum ersten Ende, wobei der Bildwandler (306, 408) mit einer zweiten linearen Rückzugsgeschwindigkeit zurückgezogen wird, die kleiner als die erste lineare Rückzugsgeschwindigkeit ist;
Abbilden des im Betrachtungsbereich (410) identifizierten interessierenden Bereichs (416), um einen Satz zweiter Bilder zu erhalten, während der Bildwandler (306, 408) von einem ersten Ende des interessierenden Bereichs (418) zu einem zweiten Ende des interessierenden Bereichs (420) entgegengesetzt zum ersten Ende zurückgezogen wird; und
Anzeigen mindestens eines Anteils des Satzes zweiter Bilder.

5. Computerlesbares Medium nach Anspruch 4, wobei die Aktionen ferner aufweisen: Positionieren des Bildwandlers (306, 408) am ersten Ende des identifizierten interessierenden Bereichs (418).

6. Computerlesbares Medium nach einem der Ansprüche 4 bis 5, wobei die Aktionen ferner aufweisen: Anzeigen mindestens eines Anteils des Satzes erster Bilder.

7. Computerlesbares Medium nach Anspruch 6, wobei die Aktionen ferner aufweisen: Markieren des identifizierten interessierenden Bereichs (416) auf dem angezeigten Satz erster Bilder.

## Revendications

1. Système d'imagerie intravasculaire (100) comprenant :
un module de commande (104) incluant un générateur d'impulsions (108), un moteur (110) et un processeur (106) ;
un cathéter (102, 402) pouvant être couplé au module de commande (104) ; et
au moins un imageur (306, 408) disposé dans le cathéter (102, 402) ;
dans lequel le processeur (106) est adapté pour exécuter des instructions lisibles par processeur pour permettre des actions, incluant :
le retrait de l'imageur (306, 408) d'une première extrémité d'une région d'étude (412) vers une seconde extrémité de la région d'étude (414) opposée à la première extrémité, l'imageur (306, 408) étant retiré à une première vitesse linéaire de retrait ;
l'imageage de la région d'étude (410) pour obtenir un ensemble de premières images tout en retirant l'imageur (306, 408) de la première extrémité de la région d'étude (412) vers la seconde extrémité de la région d'étude (414) ;
l'identification d'une région d'intérêt (416) au sein de la région d'étude (410) au moyen d'au moins une partie de l'ensemble de premières images de la région d'étude ;
le retrait de l'imageur (306, 408) d'une première extrémité de la région d'intérêt (418) vers une seconde extrémité de la région d'intérêt (420) opposée à la première extrémité, l'imageur (306, 408) étant retiré à une seconde vitesse linéaire de retrait qui est inférieure à la première vitesse linéaire de retrait ;
l'imageage de la région d'intérêt (416) identifiée au sein de la région d'étude (410) pour obtenir un ensemble de secondes images tout en retirant l'imageur (306, 408) d'une première extrémité de la région d'intérêt (418) vers une seconde extrémité de la région d'intérêt (420) opposée à la première extrémité ; et
l'affichage d'au moins une partie de l'ensemble de secondes images.

2. Système d'imagerie intravasculaire (100) selon la revendication 1, dans lequel les actions comprennent en outre le positionnement de l'imageur (306, 408) à la première extrémité de la région d'intérêt (418) identifiée.

3. Système d'imagerie intravasculaire (100) selon l'une quelconque des revendications 1 et 2, dans lequel les actions comprennent en outre l'affichage d'au moins une partie de l'ensemble de premières images.

4. Support lisible par ordinateur ayant des instructions exécutables par processeur pour imager un tissu et commander au moins une d'une vitesse de rotation, d'une vitesse ou d'une longueur de retrait d'un imageur (306, 408), les instructions exécutables par processeur lorsqu'elles sont installées sur un système d'imagerie intravasculaire (100) selon la revendication 1 permettent au système d'effectuer des actions, comprenant :
le retrait d'un imageur (306, 408) d'une première extrémité d'une région d'étude (412) vers une seconde extrémité de la région d'étude (414) opposée à la première extrémité, l'imageur (306, 408) étant retiré à une première vitesse linéaire de retrait ;
l'imageage d'une région d'étude (410) avec l'imageur (306, 408) pour obtenir un ensemble de premières images tout en retirant l'imageur (306, 408) de la première extrémité de la région d'étude (412) vers la seconde extrémité de la région d'étude (414) ;
l'identification d'une région d'intérêt (416) au sein de la région d'étude (410) au moyen d'au moins une partie de l'ensemble de premières images de la région d'étude ;
le retrait de l'imageur (306, 408) d'une première extrémité de la région d'intérêt (418) vers une seconde extrémité de la région d'intérêt (420) opposée à la première extrémité, l'imageur (306, 408) étant retiré à une seconde vitesse linéaire de retrait qui est inférieure à la première vitesse linéaire de retrait ;
l'imageage de la région d'intérêt (416) identifiée au sein de la région d'étude (410) pour obtenir un ensemble de secondes images tout en retirant l'imageur (306, 408) d'une première extrémité de la région d'intérêt (418) vers une seconde extrémité de la région d'intérêt (420) opposée à la première extrémité ; et
l'affichage d'au moins une partie de l'ensemble de secondes images.

5. Support lisible par ordinateur selon la revendication 4, dans lequel les actions comprennent en outre le positionnement de l'imageur (306, 408) à la première extrémité de la région d'intérêt (418) identifiée.

6. Support lisible par ordinateur selon l'une quelconque des revendications 4 et 5, dans lequel les actions comprennent en outre l'affichage d'au moins une partie de l'ensemble de premières images.

7. Support lisible par ordinateur selon la revendication 6, dans lequel les actions comprennent en outre le marquage de la région d'intérêt (416) identifiée sur l'ensemble de premières images affiché.
